# EUROPEAN PATENT APPLICATION

(11) **EP 3 683 310 A1**
(43) Date of publication of application: **22.07.2020**
(21) Application number: 19152890.0
(22) Date of filing: 21.01.2019
(51) Int. Cl.: C12N 15/115, C12N 15/10, C12Q 1/6811

(54) **APTAMER AND USE OF THE APTAMER IN THE DIAGNOSIS AND TREATMENT OF CANCER**

(71) Applicant: Rheinische Friedrich-Wilhelms-Universität Bonn, 53113 Bonn (DE)
(72) Inventor: DUCONGÉ, Frédéric, 92330 Sceaux (FR); LINGNAU, Andreas, 87719 Mindelheim (DE); WEBER, Holger, 79115 Freiburg (DE); KUBBUTAT, Michael, 79227 Schallstadt (DE); MAYER, Günter, 53123 Bonn (DE)
(74) Representative: Michalski Hüttermann & Partner Patentanwälte mbB

(57) **Abstract**

The present invention relates to an aptamer comprising a nucleotide sequence SEQ ID NO: 1 or a fragment thereof of at least 20 contiguous nucleotides of the nucleotide sequence SEQ ID NO: 2, wherein the aptamer comprises a polyethylene glycol (PEG) moiety conjugated to the 5' or the 3' end. The invention further relates to a composition comprising the aptamer, and the use of the aptamer in the diagnosis and treatment of cancer, particularly hormone refractory prostate tumours.

SEQUENCE LISTING

<110>  Rheinische Friedrich-Wilhelms Universität Bonn

<120>  Aptamer and use of the aptamer in the diagnosis and treatment of
       cancer

<130>  UD 41176/SAM

<160>  7

<170>  PatentIn version 3.3

<210>  1
<211>  80
<212>  DNA
<213>  Artificial

<220>
<223>  aptamer

<400>  1
gctgtgtgac tcctgcaagg aaagagcacg gccaagtcag ggggaatcga ctacgtcggg     60

gggagacaag atacagctgc                                                 80


<210>  2
<211>  43
<212>  DNA
<213>  Artificial

<220>
<223>  aptamer

<400>  2
ggaaagagca cggccaagtc agggggaatc gactacgtcg ggg                       43


<210>  3
<211>  18
<212>  DNA
<213>  Artificial

<220>
<223>  forward primer

<400>  3
gctgtgtgac tcctgcaa                                                   18


<210>  4
<211>  19
<212>  DNA
<213>  Artificial

<220>
<223>  reverse primer

<400>  4
ggagacaaga tacagctgc                                                  19

<210>  5
<211>  32
<212>  DNA
<213>  Artificial

<220>
<223>  aptamer

<400>  5
gctgtgtgac tcctgcaagg aaagagcacg gc                                    32


<210>  6
<211>  43
<212>  DNA
<213>  Artificial

<220>
<223>  aptamer

<400>  6
ggagacgcat agcagacaag actttaacgg gggccattct gtg                        43


<210>  7
<211>  43
<212>  DNA
<213>  Artificial

<220>
<223>  aptamer

<400>  7
ggatcatacc ggccgagatg cgatgcgacg tgacgtagtt cag                        43

## Description

The invention relates generally to the field of nucleic acids and more particularly to aptamers capable of binding to cancer cells.

Prostate cancer is the most common cancer among men. Especially, hormone refractory prostate cancer is not curable and targeted therapies are yet not available. Hormone refractory prostate tumour cells likely metastasise to distal sites accounting for poor prognosis and survival of patients.

Aptamers are an emerging class of molecules for developing targeted therapy approaches. Aptamers are single chained nucleic acids, folding into well-defined three-dimensional shapes based on which they recognise target structures with high affinity and specificity. Aptamers of either single-stranded DNA or RNA that specifically bind to a target ligand are produced by a technique denoted Systematic Evolution of Ligands by Exponential Enrichment (SELEX). The SELEX method is for example described by C. Tuerk and L. Gold "Systematic Evolution of Ligands by Exponential Enrichment: RNA Ligands to bacteriophage T4 DNA polymerase", Science 1990, 249, 505-510. In the SELEX method, a candidate mixture of single stranded nucleic acids having regions of randomized sequence is contacted with a target structure and those nucleic acids having an increased affinity to the target are selected and amplified. After several iterations a nucleic acid with high affinity to the target is obtained.

Aptamers targeting tumour cells are commonly identified by an *in vitro* selection process using cultured cell lines or isolated membrane proteins. However, only a few examples are described in which these aptamers are also capable of recognising the respective target or cells in the related *in vivo* microenvironment. Nuclease-mediated degradation and rapid renal filtration of nucleic acids classifies the *in vivo* selection of aptamers highly challenging. To date, a few examples of *in vivo* selected aptamers are described employing 2'-deoxy-2'-fluoro pyrimidine (2'-fluoro)-modified RNA molecules and more recently, with a phosphorothioate containing DNA library. 2'-fluoro RNA is considered to be more resistant towards nuclease hydrolysis and less prone to elicit innate immune responses. However, 2'-fluoro RNA holds a series of disadvantages, *i.e*. very cost demanding and, thus, more difficult to engineer and optimise. In addition, 2'-fluoro RNA aptamers extending 50 nucleotides in length are still difficult to synthesise chemically and in larger quantities, as required for therapeutic approaches. These characteristics make 2'-fluoro RNA challenging to be employed for basic research and drug development.

Alternatively, DNA is affordable but branded as instable and to evoke immune responses. The kidneys represent the major clearance pathway of DNA aptamers *in vivo.* A rapid renal clearance of DNA aptamers reduces their half-life and circulation time, thereby limiting their therapeutic effectiveness. The addition of a soluble polymer or carbohydrate has been shown to prevent degradation and increase the aptamer half-life. For example, PEGylation of a nucleic acid aptamer capable of binding programmed cell death protein 1 is described in WO 2016/019270 A1. The PEGylation process attaches repeating units of polyethylene glycol (PEG) to an aptamer. PEGylation can lead to increased half-life *in vivo* and lengthen residence times of aptamers in circulation, thereby decreasing dosing frequency and enhancing effectiveness.

Aptamers recognizing specific tumour cell lines have been identified, and some of them have been proven to be promising tools for diagnosis but also for the targeted delivery of potential drugs. The targeting of specific tissue is a major challenge for the effective use of therapeutics and agents mediating this targeting are demanded.

Therefore, the object underlying the present invention was to provide aptamers that recognize tumour cells.

The problem is solved by an aptamer, wherein the aptamer comprises a nucleotide sequence as given as follows: 5'-GCTGTGTGACTCCTGCAAGGAAAGAGCACGGCCAAGTCAGGGGGAATCGACTACGTCGG GGGGAGACAAGATACAGCTGC-3' (SEQ ID NO: 1) or a fragment thereof of at least 20 contiguous nucleotides of the nucleotide sequence as given as follows: 5'-GGAAAGAGCACGGCCAAGTCAGGGGGAATCGACTACGTCGGGG-3' (SEQ ID NO: 2), wherein the aptamer comprises a polyethylene glycol (PEG) moiety conjugated to the 5' or the 3' end.

The problem is also solved by a selection method that enables the *de novo* identification of pegylated DNA aptamers according to claim 14.

Surprisingly, a pegylated DNA aptamer could be selected that recognises specific tumour types, and particularly pursues tissue sites harbouring a hormone refractory prostate tumour. It could be shown that the aptamer is a tumour targeting aptamer with good specificity.The aptamer thus represents a valuable and cost-effective tool for the development of targeted therapies particularly for prostate cancer. Aptamers can be synthesised chemically and thus meet the requirements for GMP ("Good Manufacturing Practice")-conform manufacture for clinical use more efficiently than antibodies that have to be produced in cell cultures. The aptamer further demonstrated a very low immunogenicity and advantageously seemed not to activate the innate immune system. The aptamer thus is assumed to be useful for the development of vehicles for the targeted delivery of anti-cancer agents in cancer treatment as well as for a diagnostic purposes.

If not stated otherwise, the term "nucleotide" refers to a ribonucleotide or a deoxyribonucleotide. The aptamer according to the invention hence can be provided in the form of a single-stranded DNA or RNA molecule. As will be obvious to a person of ordinary skills in the art, if the nucleic acid is an RNA molecule the thymidine or "T" in the nucleotide sequence is to be read as meaning "U" or uridine. Preferably, the aptamer comprises a deoxyribonucleotide sequence. DNA aptamers can exhibit better stability. The nucleotides may comprise a chemical modification such as a locked nucleic acid (LNA). Preferred substituents are selected from the group comprising fluorine, C₁-C₅-alkoxy particularly methoxy, or an amino group.

It is assumed that even if the nucleotide sequence of the aptamer comprises an addition, deletion or substitution of one or several nucleotides it will continue to show its recognition properties. For example, the aptamer may preserve its recognition properties albeit portions of the 5'- and/or the 3'-terminal sequence of SEQ ID NO: 1 are deleted. An aptamers comprising the nucleotide sequence SEQ ID NO: 2 or a nucleotide sequence of at least 20, 25, 30 or 35 contiguous nucleotides of SEQ ID NO: 2, and optionally the 5'- and/or the 3'-terminus of SEQ ID NO: 1 or a portion thereof, may preserve its recognition properties. Hence, also aptamers comprising the nucleotide sequence SEQ ID NO: 2 or a nucleotide sequence of at least 20, 25, 30 or 35 contiguous nucleotides of SEQ ID NO: 2, or aptamers comprising the nucleotide sequence SEQ ID NO: 2 or at least 20, 25, 30 or 35 contiguous nucleotides of SEQ ID NO: 2 and the 5'- or the 3'-terminal sequence of SEQ ID NO: 1 or portions thereof, as well as pharmaceutically acceptable salts thereof are part of the present invention. Preferred aptamers are the aptamers of SEQ ID NOs: 1 and 2.

The aptamer comprises a polyethylene glycol (PEG) moiety conjugated to the 5' end or the 3' end. The aptamer may be conjugated to polyethylene glycol (PEG) moieties via covalent linkage, non-covalent linkage, or both. Methods for manufacturing pegylated oligonucleotides and PEGylation reagents of defined numbers of PEG units are known in the art. The PEG moiety conjugated to the aptamer can be linear or branched. It may be conjugated to the 5' end of the nucleic acid aptamer, the 3' end of the aptamer, or both. In embodiments, the aptamer comprises a 5'-polyethylene glycol (PEG) moiety. The PEG moiety can be covalently conjugated to the 5' end of the nucleic acid aptamer. Polyethylene glycol is a biologically inert, non-immunogenic compound, and is non-toxic and highly flexible.

Without being bound to a specific theory, it is assumed that the aptamer's cell binding properties depend on the presence of the PEG moiety. Spectroscopic data indicate that the conformation of the DNA aptamer of SEQ ID NO: 1 is not impaired by the PEG moiety and that the PEG moiety seems not to have much impact on the aptamer's folding. Thus, it is assumed that the PEG moiety most likely directly interacts with the target structure of the cell surface.

In embodiments, the polyethylene glycol (PEG) moiety has a molecular weight in a range of ≥ 0.1 kDa to ≤ 90 kDa, preferably in a range of ≥ 2 kDa to ≤ 20 kDa, or in a range of ≥ 2 kDa to ≤ 11 kDa. The PEG moiety advantageously increases the half-life of DNA molecules in the blood circulation. It was shown that a 5'-PEG moiety of 2 kDa provided a higher binding ratio of the aptamers compared to PEG moieties of higher weight. On the other hand, the addition of a 11 kDa PEG moiety to the aptamer of SEQ ID NO: 1 increases the molecular weight to about 36 kDa, which is in the range of the renal glomerulus threshold. Particularly, a molecular weight of the PEG moiety in a range of 2 kDa to 11 kDa can combine a very good binding ratio with improved ability.

A further aspect of the present invention relates to an aptamer, wherein the aptamer comprises a nucleotide sequence as given as follows: 5'-GCTGTGTGACTCCTGCAAGGAAAGAGCACGGCCAAGTCAGGGGGAATCGACTACGTCGG GGGGAGACAAGATACAGCTGC-3' (SEQ ID NO: 1) or a fragment thereof of at least 20, 25, 30 or 35 contiguous nucleotides of the nucleotide sequence as given as follows: 5'-GGAAAGAGCACGGCCAAGTCAGGGGGAATCGACTACGTCGGGG-3' (SEQ ID NO: 2), wherein the aptamer comprises a polyethylene glycol (PEG) moiety conjugated to the 5' or the 3' end for use as a medicament or a diagnostic reagent.

The aptamer may comprise a 5'-polyethylene glycol (PEG) moiety. The PEG moiety may have a molecular weight in a range of ≥ 0.1 kDa to ≤ 90 kDa, preferably in a range of ≥ 2 kDa to ≤ 20 kDa, particularly in a range of ≥ 2 kDa to ≤ 11 kDa.

The aptamer advantageously recognises cancer cells *in vivo* and *in vitro.* Further, the aptamer showed consistent tumour targeting in orthotopic and subcutaneous xenograft mouse models. The aptamer was found to reproducibly recognise prostate tumours *in vivo.* This provides a hugh advantage, as targeting of tumour tissue in its native environment is a major challenge in cancer therapy in general and in particular for respective organs that are difficult to treat, e.g. prostate. The aptamer particularly recognised specific tumour types, and particularly tissue sites harbouring a hormone refractory prostate tumour. Further, the aptamer was found to interact with the lung cancer cell lines A549 and H460 (lung carcinoma), while no binding to splenocytes, PMBCs, Ramos (Burkitt's lymphoma), and MCF7 (breast cancer) cells was observed. The aptamer thus provides high specificity towards prostate and lung cancer cells. Hence, the aptamer represents a promising tools for cancer therapy and diagnosis. Preferably, the aptamer is capable of binding specifically to prostate and lung cancer cells.

A further aspect of the present invention relates to an aptamer, wherein the aptamer comprises a nucleotide sequence as given as follows: 5'-GCTGTGTGACTCCTGCAAGGAAAGAGCACGGCCAAGTCAGGGGGAATCGACTACGTCGG GGGGAGACAAGATACAGCTGC-3' (SEQ ID NO: 1) or a fragment thereof of at least 20, 25, 30 or 35 contiguous nucleotides of the nucleotide sequence as given as follows: 5'-GGAAAGAGCACGGCCAAGTCAGGGGGAATCGACTACGTCGGGG-3' (SEQ ID NO: 2), wherein the aptamer comprises a polyethylene glycol (PEG) moiety conjugated to the 5' or the 3' end for use in the detection, diagnosis or treatment of cancer.

It was found that the aptamer specifically detects and bind to cancer cells, particularly to prostate and lung cancer cells. This suggests that the aptamer according to the invention can be used for the detection or diagnosis of cancer. Aptamers are usable for carrying toxins or siRNA molecules influencing tumour cell viability. Hence, the aptamer is useful as a medicament or a diagnostic reagent.

In embodiments, the cancer is selected from lung cancer and prostate cancer, particularly hormone refractory prostate cancer. The aptamer specifically recognises prostate cancer cells and lung cancer cells. Advantageously, the aptamer specifically is usable as a medicament or a diagnostic reagent for lung cancer and prostate cancer, particularly for hormone refractory prostate cancer.

The aptamer may comprise a 5'-polyethylene glycol (PEG) moiety. The PEG moiety may have a molecular weight in a range of ≥ 0.1 kDa to ≤ 90 kDa, preferably in a range of ≥ 2 kDa to ≤ 20 kDa, or in a range of ≥ 2 kDa to ≤ 11 kDa.

The aptamers according to the present invention also are usable in form of pharmaceutically acceptable salts thereof. The term "pharmaceutically acceptable salts" refers to salts prepared from pharmaceutically acceptable non-toxic bases or acids. A pharmaceutically acceptable salt can be conveniently prepared from pharmaceutically acceptable non-toxic bases, including inorganic bases and organic bases. Preferred salts derived from inorganic bases include ammonium, calcium, magnesium, potassium and sodium salts. Salts derived from pharmaceutically acceptable organic non-toxic bases include salts of primary, secondary, and tertiary amines, as well as cyclic amines. Preferably, the pharmaceutically acceptable salt is selected from the group of sodium or potassium salts. Also, calcium or magnesium salts can be preferred.

For use as a medicament or a diagnostic reagent the aptamers can be used or included in a composition. In embodiments, the composition comprising an aptamer according to the invention is usable in the detection or diagnosis, or the treatment of cancer, particularly prostate cancer and lung cancer. For use as a diagnostic reagent the aptamers can be used or included in a diagnostic composition. For use as a medicament the aptamers can be used or included in a pharmaceutical composition.

Accordingly, in another aspect the present invention relates to a composition comprising an aptamer according to the invention as described above. The composition particularly is usable in the detection or diagnosis of cancer, particularly prostate cancer and lung cancer, especially hormone refractory prostate cancer.

When the aptamer is brought into contact with a sample, it will bind specifically to a cancerous cell present in the sample. For diagnostic purposes, the aptamer may comprise a labelling which provides that the bound aptamer can be detected by determining the presence or absence of a signal provided by the label. For example, the aptamer can be labelled with a fluorescent dye. A fluorescence-labelling, for example provided by a fluorescence dye, can provide a visualisation of the bound aptamer by fluorescence or laser scanning microscopy or flow cytometry. Further, particularly for detection purposes, the aptamer can be immobilised on conventional supports such as beads providing a tool for the detection of aptamer-bound cancerous cells and thus diagnosing cancer. Further, the aptamer can be biotinylated or coupled to streptavidin, avidin or neutravidin for use in the specific detection of tumour cells.

Besides being useful for detecting or diagnosing cancer by recognising tumour cells, the aptamers also are applicable for targeted therapies. In another aspect the present invention as described above relates to a pharmaceutical composition comprising an aptamer according to the invention as an active ingredient. The aptamers particularly are usable as a vehicle for delivery of cargo therapeutics, such as anti-cancer drugs, toxins or antagomirs to prostate tumours.

Antagomirs can be attached to the aptamers to be delivered to tumour cells. As used herein, the term "antagomir" refers to an oligonucleotide that can bind to microRNA (miRNA). Antagomirs are usable to affect the regulation of microRNA molecules or inhibit or silence endogenous microRNA. For example, antagomirs against microRNA 155 or miR21 induce a knock-down resulting in an inhibition of tumour growth. Also, aptamer-antagomir conjugates thus are usable in aptamer-targeted cell-specific RNA interference in tumour cells.

Especially cancer-specific drugs or toxins inducing apoptosis or cell death can be attached to the aptamers to be delivered to tumour cells. Cancer-specific drugs for treatment of hormone refractory prostate tumour may be selected from paclitaxel, docetaxel (taxotere), sipuleucel-T, abiraterone, enzalutamide, and radiopharmaceuticals. For prostate tumours not responding to initial hormone therapy, chemotherapy with docetaxel (taxotere) is used. Other therapies are immunotherapy with sipuleucel-T, an autologous dendritic cell- based cancer vaccine, agents interfering with androgen signalling, i.e. abiraterone or enzalutamide, and radiopharmaceutical therapy for bone metastases. The aptamer particularly may function as vehicle for the specific delivery of cargo therapeutics, e.g. paclitaxel or antagomirs to prostate tumours.

The aptamer particularly may also function as vehicle for the delivery of siRNA (small interfering RNA) molecules into tumour cells. Hence, the pharmaceutical composition the aptamers can particularly be useful as a molecular vehicle for delivery of cargo such as siRNA molecules into the cells leading to a deprivation of tumour cells. The cargo molecules such as anti-cancer drugs, toxins, siRNA or antagomir molecules can be directly coupled to the aptamer, in a covalent or non-covalent fashion. Alternatively, the aptamer can be attached to the surface of a liposome containing an anticancer agent.

In embodiments, the composition or the pharmaceutical composition, respectively, is for use in the detection or diagnosis, or in the treatment of cancer. The cancer may be selected from lung cancer and prostate cancer, particularly hormone refractory prostate cancer.

A composition comprising an aptamer according to the invention, particularly a pharmaceutical composition for use in the treatment of cancer, particularly prostate tumours or lung cancer, can further comprise an anti-cancer agent such as a toxin, anti-cancer drugs, an antagomir or siRNA, particularly for hormone refractory prostate cancer or lung cancer-specific delivery of therapeutics. Accordingly, in a further aspect the present invention relates to a cancer-specific drug delivery composition comprising an aptamer according to the invention, and an anti-cancer agent such as a toxin, an anti-cancer growth inhibitor gene, an antagomir, siRNA, or combinations thereof. The cancer-specific drug delivery composition may be specific for lung or prostate cancer particularly for hormone refractory prostate cancer.

The compositions particularly the pharmaceutical composition may be produced under sterile conditions using standard pharmaceutical techniques well known to those skilled in the art. For compositions convenient pharmaceutical media may be employed. For example, water, glycols, oils, alcohols and the like may be used to form liquid preparations such as solutions. The composition may comprise a pharmaceutical carrier, which can be, for example, a solid, liquid, or gas. Suitable carriers preferably are liquid and correspond to the substances ordinarily employed in formulation technology for pharmaceutical formulations. The compositions can be suitable for parenteral administration, including subcutaneous, intramuscular, and intravenous administration. Compositions suitable for parenteral administration may be prepared as solutions or suspensions of the aptamer in water. Compositions suitable for injectable use include sterile aqueous solutions or dispersions.

The present invention also relates to the use of an aptamer comprising a nucleotide sequence as given as follows: 5'-GCTGTGTGACTCCTGCAAGGAAAGAGCACGGCCAAGTCAGGGGGAATCGACTACGTCGG GGGGAGACAAGATACAGCTGC-3' (SEQ ID NO: 1) or a fragment thereof of at least 20, 25, 30 or 35 contiguous nucleotides of the nucleotide sequence as given as follows: 5'-GGAAAGAGCACGGCCAAGTCAGGGGGAATCGACTACGTCGGGG-3' (SEQ ID NO: 2), wherein the aptamer comprises a polyethylene glycol (PEG) moiety conjugated to the 5' or the 3' end, for the manufacture of a medicament or a diagnostic reagent. The medicament and the diagnostic reagent preferably are for the detection or diagnosis or the treatment of cancer, preferably a cancer selected from lung cancer and prostate cancer, particularly for the detection or diagnosis or the treatment of hormone refractory prostate cancer.

A further aspect of the present invention relates to an *in vitro* method of detecting or diagnosing cancer, particularly prostate cancer, the method comprising the step of detecting the binding of an aptamer according to the invention to a cell, tissue, or sample obtained from a subject.

As used herein, the term "sample" refers to any material, which probably contains tumour cells, including any liquid or fluid sample or solid material, particularly a sample derived from a biological source such as a patient or test subject. The term sample particularly refers to biological material, for example cells or tissues, biological fluids or supernatants. The biological material can be a tissue specimen removed from a cancer subject, preferably humans, for example, by surgical resection or biopsy. The biological material can be a body fluid such as blood, serum, plasma, saliva, phlegm and urine. The method comprises bringing the aptamer into contact with a sample, which probably contains tumour cells. The sample may be derived from a biological source such as a cancer subject. The sample for example can comprise cells or a tissue specimen isolated from a cancer subject, preferably a human, for example, by surgical resection or biopsy. The sample also can be a body fluid such as blood, serum, plasma, saliva, phlegm and urine.

A further aspect of the present invention relates to a method of treating cancer, the method comprising the step of administering to a subject a therapeutically effective amount of an an aptamer or a pharmaceutical composition according to the invention, or a cancer-specific drug delivery composition according to the invention. The cancer may be lung cancer or prostate cancer, particularly hormone refractory prostate cancer. The term "therapeutically effective amount" is used herein to mean an amount or dose sufficient to cause an improvement in a clinically significant condition in the subject.

A further aspect of the present invention relates to a method for selecting aptamers specific for a target sample, the method comprising the steps:
- providing a library comprising putative disease-specific aptamers;
- incubating the library with a target sample;
- washing the target sample to remove aptamers that are not bound;
- eluting the aptamers bound to the target sample,
- amplifying the eluted aptamers using polymerase chain reaction to generate an enriched pool of aptamers;
- repeating the incubating, washing, eluting and amplification steps a plurality of additional times to generate a pool of enriched aptamers that are specific for the target sample, and
- sequencing the pool of enriched aptamers to determine their sequence and relative frequency within the pool,
wherein the aptamers of the library comprise a conjugated polyethylene glycol (PEG) moiety.

Surprisingly, a successful selection of aptamers from a pegylated DNA library could be achieved. Regarding the aptamers comprising a conjugated polyethylene glycol (PEG) moiety reference is made to the description above.

The SELEX concept can be used for *in vitro* selection using cell cultures or tissue samples, or can be extended to *in vivo* selection methods. The target sample may be a tissue sample affected with a disease, particularly a cancer tissue or a cancer cell line, such as the prostate cancer cell line PC3. In an embodiment, the method is an *in vivo* method, wherein the target sample is a tumour tissue provided in an *in vivo* model. The *in vivo* model preferably is a murine orthotopic xenograft model. Advantageously, murine orthotopic xenograft models are compatible with *in vivo* selection experiments. As these model systems reflect the natural microenvironment of tumours in cancer biology very well, the resultant aptamers may bear great potential to be further developed as targeted therapeutic regimen. The method also opens the path towards applicable orthotopic model systems, e.g. melanoma, breast cancer, colon cancer, pancreatic cancer, non-small cell lung cancer, and even metastasis model systems, with which the method is compatible. The method provides the benefit of combining *in vivo* selection with high-throughput sequencing and thorough data analysis.

After orthotopic tumour implatation and growth, an *in vivo* model such as a murine orthotopic xenograft model can be provided. The target sample may be a tissue affected with cancer such as a prostate tumour. The library can be injected intravenously. For incubation of the library with the tumour tissue, a circulation of the oligonucleotides for a certain time period such as 20 minutes may be provided. The washing step to remove aptamers that are not bound to the tumour tissue may be provided by an *in vivo* perfusion, for example with a buffer. After perfusion, the tumour tissue and a control tissue are collected. A usable control tissue in an *in vivo* model is, for example, another organ such as kidney tissue. For eluting, in an *in vivo* model such as a murine orthotopic xenograft model, the bound aptamers are extracted. In a following step, the extracted aptamers are amplified. A following single strand displacement of the purified PCR product may be carried out by exonuclease digestion. The enriched pool of aptamers provides a first next-generation library for the next selection round. The cycles of incubating, perfusion, collecting, elution or extracting and amplification may be repeated for a plurality of additional times, for example for 8 to 12 or 10 cycles, to generate a pool of enriched aptamers that are specific for the target sample such as a tumour tissue. Usually, the incubation of the first next-generation library during the second and following selection cycles employs a second set of conditions that are more stringent than the first set of conditions. After completion of the cycles the pools of enriched aptamers are sequenced to determine their sequence and relative frequency. In embodiments, the sequencing comprises next generation sequencing (NGS). For identifying relevant sequences a testing in an *in vivo* screening may be performed. Advantageously, a pegylated DNA library was found to be sucessfully employed in an *in vivo* murine orthotopic xenograft model.

Unless otherwise defined, the technical and scientific terms used herein have the same meaning as commonly understood by one of ordinary skill in the art to which this invention belongs.

The Examples, which follow serve to illustrate the invention in more detail but do not constitute a limitation thereof.

The figures show:
- Figure 1: The *in vivo* selection scheme for the aptamer of SEQ NO: 1.
- Figure 2: Analysis of the number of unique sequences within the obtained libraries from the *in vivo* selection using D3 (squares) and D3P (dots).
- Figure 3: The validation of aptamers evolved from the DNA library D3P by planar imaging.
- Figure 4: Quantification of tumour binding of the aptamer of SEQ NO: 1 (D3P-21) *in vitro.* The figure 4a shows the relative amount of the aptamer and native library (D3P-lib) used as control of tumours from subcutaneous mouse models, figure 4b the relative amount of aptamer from both orthotopic and subcutaneous tumours, both quantified via qPCR.
- Figure 5: The stability of the pegylated (figure 5a) and non-pegylated (figure 5b) aptamer of SEQ NO: 1 (D3P-21) in human serum compared to DPBS with Ca²⁺/Mg²⁺ incubated in human serum (quadrants) or buffer (cycles).
- Figure 6: The flow cytometry analysis of the interaction of the aptamer of SEQ NO: 1 (D3P-21) with various cancer cell lines.
- Figure 7: The flow cytometry analysis of the impact of the PEG moiety of the aptamer of SEQ NO: 1 (D3P-21) on its interaction properties.
- Figure 8: Impact of potassium ions on the interaction of the aptamer of SEQ NO: 1 with PC-3 cells.
- Figure 9: CD spectroscopy analysis of the aptamer of SEQ NO: 1 (D3P-21).
- Figure 10: The evaluation of aptamer immunogenicity.
- Figure 11: The effect of the molecular weight of the PEG moiety on binding.

### Material and Methods

### Orthotopic PC-3 tumour model:

On Day 0, 3x10⁶ PC-3 tumour cells expressing luciferase in 15µl PBS were implanted orthotopically into 20 male NMRI nude mice. To prevent pain, Meloxicam (Metacam, 1mg/kg, s.c.) was applied 1 h prior to surgery and 24 h post implantation. Male NMRI nude mice were anesthetised in a separate box using 1.5-2 Vol% Isoflurane with an oxygen flow of 0.6 l/min. The mice were positioned on a heated operating table with the left side upwards. The skin was cleaned, shaved and sterilised. An incision of approx. 1 cm was made in order to display the seminal vesicle and the prostate. A cell suspension of 3x10⁶ PC-3Luc cells in 15µl PBS was injected orthotopically into the prostate using a 29G needle syringe. The seminal vesicle and the prostate were carefully pushed back into the visceral cavity and the abdominal wall closed by saturation. Thereafter the mouse was warmed in a separate box while recovering from anaesthesia. In the following, animal weights were measured three times weekly (Monday, Wednesday and Friday).

### Subcutaneous PC-3 tumour model:

For in vivo screening experiments, mice were subcutaneously injected between shoulder blades with 3x10⁶ PC-3 cells in a volume of 200 ml of Matrigel (BD Bioscience, Le Pont de Claix, France) and phosphate- buffered saline (PBS) (50:50). Tumours were then allowed to grow for 3-5 weeks until a size around 300 mm3 before in vivo imaging experiments. During each injection and imaging experiments, mice were anesthetised with isoflurane-1.25% in a 1:3 mixture of O₂ and air. Subcutaneous injection were performed 3 weeks after orthotopical implantation as previously described or in mice without orthotopic implantation.

### In vivo bioluminescence imaging:

During the course of the study, tumour growth was monitored *in vivo* using bioluminescence imaging. For this purpose, 150 mg/kg D-Luciferin was injected intraperitoneally (i.p.) into the mice 7 min before anesthetisation. Light emission was measured 10 min post injection with a CCD-camera for 5 min using a NightOWL LB 981 bioluminescence imaging system (Berthold Technologies, Germany).

### In vivo SELEX:

All oligonucleotides, including DNA libraries and primers, were synthesised by Ella Biotech GmbH (Munich, Germany). Two separate 80-nt single-stranded DNA libraries, D3 and D3P, were used consisting in a 43-nt random region. The D3P library contained an 11kDa polyethylene glycol (PEG) moiety on the 5'-end. The D3 library did not contain a PEG moiety. Both libraries were amplified by PCR using the following primers: forward primer (Fw) 5'-GCTGTGTGACTCCTGCAA-3' (SEQ ID NO: 3), with 5'-11kDa PEG moiety in the case of D3P library, and reverse primer (Rv-Pho) 5'-Phosphate-GGAGACAAGATACAGCTGC-3' (SEQ ID NO: 4). PCR reaction was performed by using GoTaq® G2 Flexi DNA Polymerase (Promega) and 1 µM of both Fw and Rv-Pho primers with the following cycling program (2 min 95°C; 30 sec 95°C, 30 sec 64°C, 45 sec 72°C; hold 10°C) in a Veriti 96 well thermal cycler (Applied Biosystems).

*In vivo* selection was done using tumour-bearing animals. Mice were either treated intravenously with one of two aptamer libraries (D3 or D3P) or left untreated. Five nmol of libraries were injected in round 1 and the amount was reduced to 2, 1 and 0.5 nmol for rounds 2, 3 and 4 respectively. From round 5 to 10, 0.1 pmol of the libraries were injected. Prior injection, libraries were prepared in 110 µL of Dulbecco's phosphate-buffered saline (DPBS) containing Ca⁺⁺ and Mg⁺⁺ (Gibco), denatured at 80°C for 3 min and slowly cooled down to RT for proper folding. After 20 min, animals were perfused with DPBS, killed by cervical dislocation and tumours and kidney were collected. Tumours and kidney were snap-frozen in liquid nitrogen and stored at -80°C before recovering the nucleic acids from the tissues. Weight of the tumours and kidneys used during *in vivo* SELEX was determined. Extraction of the nucleic acids from 3 tumours and 3 kidneys from injected mice was performed by first homogenising the organs with a 7 mL dounce tissue grinder with large and small clearance pistils (Landgraf Laborsysteme HLL GmbH). To lysate the cells, TE-SDS Lysis buffer (0.1 M Tris-HCl pH 8, 1 mM EDTA pH 8, 0.5% SDS) supplemented with 0.5 µg/µL of Proteinase K (Roth) was used for D3 injected tumours or kidney, followed by 10 min incubation at 95°C.

For D3P library, buffers A1, A2 and A3 from NucleoSpin® Plasmid kit (Macherey-Nagel) were used for lysis of the cells followed by 10 min centrifugation at 4000 rcf to remove cellular debris. Purification of extracted oligonucleotides was performed by means of phenol/chloroform extraction and ethanol precipitation for D3 library, and by silica columns (DNA Clean & Concentrator™ -500 (Zymo Research)) in the case of D3P library. A negative control was always included with tumours extracted from control mice (injected with DPBS) following the same procedure described above. Purified oligonucleotides were then re-dissolved in milliQ water and a first PCR amplification was performed. RNA digestion was performed before PCR amplification by using a 1:1 mixture of RNase T (Roche) and RNAse A (Macharey-Nagel). Agarose gel (4%) purification was then performed in order to separate the library band from genomic DNA and primers with the NucleoSpin® Clean-Up kit. Further PCR amplification was then performed to reach the required amount of library the next round. All tissue homogenisations and PCR preparations were performed in two different PCR workstations (Peqlab) in order to avoid contaminations. Single strand displacement of the purified PCR product was carried out by λ-exonuclease digestion in IX λ-exonuclease reaction buffer and 5000 U/mL of λ-exonuclease (Thermo Scientific). After 30 min incubation at 37°C, λ-exonuclease was inactivated at 80°C for 10 min. Subsequently the samples were purified with the NucleoSpin® Clean-Up kit using the NTC buffer and resulting DNA libraries were freeze dried and frozen prior usage for next selection round.

### Next Generation Sequencing:

After 10 selection rounds, samples from all rounds for both tumour and kidney of the two DNA libraries were prepared for next generation sequencing (NGS) analysis on Illumina HiSeq1500 platform following the protocol from Tolle et al. in Nucleic Acid Aptamers: Selection, Characterization, and Application, G. Mayer, Editor. 2016, Springer New York: New York, NY. p. 77-84. Shortly, a first PCR with index containing primers was performed. Those indexes allow the analysis of 12 different samples on the same round. After purification of the PCR product as described above, up to 12 different samples with different indexes were mixed with equal amounts of DNA, to a final amount of 2 µg DNA. Then, addition of adapter sequences by enzymatic ligation was performed according to the manufacturer by using TruSeq DNA PCR-Free Sample Preparation Kit LT (Illumina), following the steps "End Repair", "Adenylation" and "Adapter Ligation". Samples were then purified via agarose gel (2%) and silica based spin-columns, and eluted in resuspension buffer. Quantitative PCR was performed for library validation with the KAPA library quantification kit (Sigma-Aldrich) prior sequencing. Seventy-five base pair single end sequencing was carried out. Raw NGS data was analysed using the COMPAS (COMmonPAtternS) software.

### In vivo Planar NIR fluorescence imaging of candidate aptamers:

Mice were housed under standard conditions with food and water ad libitum but using chlorophyll free diet 15 days before imaging in order to reduce autofluorescence signal of the animals. Imaging experiments and analysis were performed using a fluorescence Diffuse Optical Tomography (fDOT) imaging system. Basically, the acquisition of Fluorescence reflectance imaging (FRI) is based on the excitation of fluorophores by the LEDs (emitting light between 650 and 670 nm) placed above the animal and on the reception of the fluorescence signal using the CCD camera and a band-pass filter (730 ± 15 nm). The CCD camera was focused at the top surface of the animal. Prior to intravenous injection and imaging of the aptamers, solutions of all sequences containing 2 nmol were prepared in DPBS with calcium and magnesium. All sequences were then heated for 3 minutes at 80°C, spin down, let to cool in ice for 3 minutes and store at room temperature. Prior to imaging, mice were anesthetised with 4 % isoflurane gas. Afterwards the level of isoflurane concentration was lowered down to 2-2.5 %. The natural auto-fluorescence of the mice was recorded just before injection and was further subtracted in order to obtain the accurate fluorescence signal from the injected fluorescent probes. Then, the fluorescent aptamers were injected in the tail vein using a 29G (insulin-type) syringe in a volume of 100 µL. Fluorescence images of dorsal side view were acquired 5 min, 90 min and 180 min post injection. From experience, good contrast is obtained after exposition times of a few milliseconds. Since aptamers are rapidly eliminated by the urinary pathway, the biodistribution of aptamers in prostate tumours could not be measured by in vivo imaging. Therefore, animals were euthanised 3 h after injection and organ resection permitted *ex vivo* fluorescence analysis of tumours and muscles.

### Planar Image analysis:

For the semi-quantitative analysis of fluorescence planar images, the ImageJ software (http://rsbweb.nih.gov/ij/) was used. The first step was to subtract the intrinsic background noise of the camera from each image acquired. Second step was to normalise the images to the same exposure time. An ROI was manually drawn, to delineate the tumour, based on the white images (photographs) that are always acquired before initialising the experiments. The mean of intensity in this region was subtracted from the mean of intensity in the same area before injection, which corresponds to the auto-fluorescence of the animal at time t₀. Using normalised images as well, a ROI was manually drawn for each time to delineate a reference healthy area close to the tumour tissue. The tumour targeting of aptamers was evaluated by dividing the mean fluorescence from the tumour by the mean fluorescence from the healthy zone. For *ex vivo* analysis, the same protocol was used and the tumour/muscle ratios were calculated.

### qPCR of tissue:

Orthotopic and xenograft tumours from mouse injected with Alexa Fluor 680 labeled D3P-21 and control sequences D3P-library, D3P-4 and D3P-16 from the *in vivo* screening were homogenised and purified as described above for D3P library. The amount of extracted DNA was quantified with NanoDrop 2000C (Thermo Scientific) and for qPCR quantification samples were normalised to a same OD. Two µL sample of the extracted DNA were added to 18 µL of a PCR master mix, containing IX GoTaq colorless buffer, 2 mM MgCl₂, 0.2 mM dNTPs, 300 nM of non-modified reverse and forward primers, IX SYBR Green I (Sigma Aldrich) and 2.5 U GoTaq polymerase. Thermal conditions were optimised to 10 min 95°C followed by 40 cycles of 30 s at 95°C, 30 s at 64°C and 45 s at 72°C. Thermal cycling was performed in an iCycler Thermal Cycler upgraded with the iQ5 real-time PCR detection system (Bio-Rad, Germany). DNA standards were included; 20 - 0.002 fmol in 1 to 10 dilution. Each sample and standard were run in duplicates.

### Cell culture:

For the *in vitro* evaluation of D3P-21 aptamer, different cell lines were used. The tumour cell line PC-3 was obtained from ProQinase, Ramos (Burkitt's lymphoma), A549 (human non-small cell lung cancer) and H460 (large cell lung cancer) were obtained from ATCC (American Type Culture Collection). MCF7 cells (breast cancer) were purchased from CLS (Cell lines service). Splenocytes and PMBC's were obtained from the spleen and the blood, respectively, of C57/BL6J mouse strain (kindly provided by Dr. Sven Burgdorf from the LIMES Institute in Bonn). Ramos, MCF7, H460 and LNCaP cells were cultured in RPMI 1640 medium while PC-3 and A549 cells were cultured in DMEM, high glucose, GlutaMAX™ supplemented (ThermoFisher) both with 10% fetal bovine serum (Sigma) at 37°C in humidified air containing 5% CO₂, and maintained by routine passage every 2-3 days. Prior usage, cells were counted with a hemacytometer. Suspension cells were centrifuged 5 min at 200 rcf and the pellet was suspended in fresh medium to obtain a cell suspension with the desired densities. For adherent cells, 100000 cells/well in appropriate medium were seeded in 24 well plates 24 hours prior the assay and proper amount of LNCaP cells were seeded in T12.5 cell culture flasks 48 hours prior the assay.

### Binding assays:

Flow cytometry assays were performed in a BD FACSCanto cytometer and qPCR assays with an iCycler Thermal Cycler upgraded with the iQ5 real-time PCR detection system (Bio-Rad, Germany).

### Flow cytometry:

For studying the interaction of D3P-21 aptamer to different cancer cell lines, 3'-Alexa Fluor 680 labeled D3P-21 and control library D3P-library were used whereas 3'-Atto647N labeled oligonucleotides were used to study the 11-kDa PEG moiety influence in the aptamer binding and the interaction of D3P-21 aptamer to both prostate cancer cell lines PC-3 and LNCaP. Cells were incubated with 100 or 200 nM of D3P-21 aptamer or D3P-library control in 200 µL of binding buffer or 750 µL for LNCaP cells (DPBS with 0.49 mM MgCl₂, 0.9 mM CaCl₂ and 0.5 mg/mL salmon sperm) for 30 minutes at 37°C and 5% CO₂. Then, cells were washed 3 times with washing buffer (DPBS with 0.49 mM MgCl₂, 0.9 mM CaCl₂) *via* centrifugation at 200g for 5 minutes at room temperature for suspension cells, and with scraping of the adherent cells in the last washing step followed by centrifugation for volume reduction. For LNCaP cells, CaCl₂ was removed from the last washing step in order to prevent clumping of the cells. For each measurement, 10000 cells were analysed in the flow cytometer. The data was analysed using FlowJo software.

### qPCR:

The same incubation protocol as used for flow cytometry analysis for the individual sequences/library was followed. After 3 washing steps, cold ddH₂0 was added and cells were incubated at 4°C for 30 min. Cells were then recovered from the well plate, heated at 95°C for 5 min, and diluted to 5 cells/µL for analysis *via* qPCR. qPCR protocol was identical to the one described above (qPCR of tissue section).

### CD Spectroscopy studies:

CD spectra were recorded at 20°C with a Jasco J-810 spectrophotometer. The measurements were performed with 8 µM of DNA oligos in water, PBS without potassium (130 mM NaCl, 7 mM Na₂HPO₄·H₂O and 3 mM NaH₂PO₄·2H₂O, pH 7.4), and increasing concentrations of KCl (0.1, 1, 4 and 10 mM). The spectra were recorded with 100 nm min-1 scanning speed and 4 accumulations.

### TNF-α homogeneous time-resolved fluorescence (HTRF) assay:

The TNF-α HTRF assay was performed in accordance with the manufacturer guidelines (Cisbio). Briefly, immortalised murine embryonic stem cell-derived macrophages in 96-well plates were treated with increasing concentrations of D3P-library and aptamers D3P-21 and D3P-20, both containing or lacking of the 5'- 11 kDa PEG moiety, for 24 hours. CpG oligonucleotide and LPS were used as positive controls. The cell supernatants were collected and stained with anti-TNF-a antibodies conjugated to FRET molecules. Changes in the fluorescence emission spectrum were proportional to the TNF-α concentration.

### Example 1

### Selection method for the aptamer of SEQ ID NO: 1

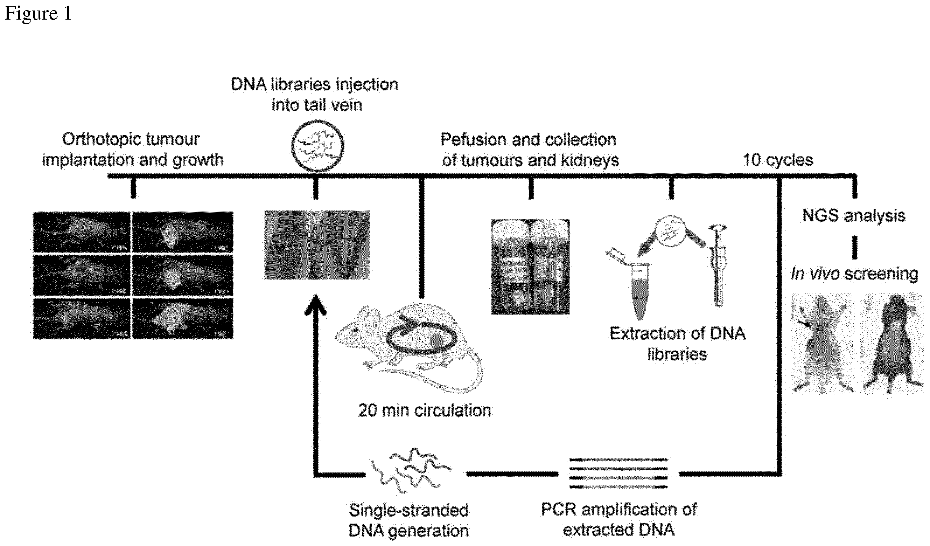

The general outline of the *in vivo* selection scheme using orthotopic xenograft prostate tumour models is shown in Figure 1. As depicted in Figure 1, the prostate cancer cell line PC-3 was implanted into the prostate of male NMRI nude mice and tumour growth was monitored by bioluminescence imaging during 5 weeks until complete growth of the tumours, before 10 selection rounds were performed. PC-3 is a human hormone insensitive prostate tumour cell line, which represents a well-accepted tumour model for castration resistant prostate cancer with respect to its sensitivity to current treatments. The used cell line bears an intrinsic luciferase reporter protein enabling post-surgery monitoring of the tumour growth.

Approx. five weeks after implantation, the respective DNA library or phosphate buffered saline (PBS) was injected into the mice's tail vein and 10 selection rounds were performed. Two *in vivo* selection protocols using the DNA library D3 were established. The first selection protocol employed D3 in its naive variant, whereas the second protocol made use of a 5'- polyethylene glycol (PEG, 11kDa) modified version, named D3P. Besides the nature of the DNA library used, both protocols differed in the work up procedure of the DNA molecules after tumour and kidney resection and homogenisation as described in the methods section above. Prior to injection, the DNA libraries were prepared as a solution in PBS. After 20 minutes of circulation, the mice were perfused with PBS and sacrificed by cervical dislocation. Subsequently, the prostate tumour was removed, snap frozen, and stored at -80°C until further processing. After thawing, the tissue was homogenised and the nucleic acid library recovered either by silica column purification (D3P) or phenol/chloroform extraction followed by ethanol precipitation (D3). After recovery, the library was amplified by PCR, subjected to single-strand generation and used for the next selection cycle. In each selection cycle, the DNA molecules associated with the kidneys were also recovered and subjected to PCR, which allowed to control the general workflow as the kidneys represent the major clearance pathway of DNA aptamers *in vivo.* As further control, tumours from mice injected with PBS only were prepared and subjected to the same recovery procedure and PCR protocol. The weight of the resected tumours and kidneys from each selection cycle was determined. The conditions of the ten *in vivo* selection cycles are summarised in Table 1 below.

**Table 1: conditions of in vivo selection cycles**

| SELEX cycle | Library inlected (nmol) | PCR amplification cycle | | | |
|---|---|---|---|---|---|
| | | D3 tumour (15 mg/PCR) | D3 kidney (15 mg/PCR) | D3P tumour (6.25 mg/PCR) | D3P kidney (6.25 mg/PCR) |
| 1 | 5 | 22 | 22 | 22 | 22 |
| 1 | 0.1 | 16 | 20 | 20 | 18 |
| 2 | 2 | 14 | 14 | 18 | 16 |
| 3 | 1 | 12 | 12 | 18 | 14 |
| 4 | 0.5 | 10 | 10 | 19 | 18 |
| 5 | 0.1 | 12 | 12 | 16 | 14 |
| 6 | 0.1 | 10 | 10 | 18 | 16 |
| 7 | 0.1 | 12* | 10* | 14 | 14 |
| 8 | 0.1 | not determined | not determined | 14 | 14 |
| 9 | 0.1 | 12* | 12* | 14 | 12 |
| 10 | 0.1 | 12* | 12* | 16 | 14 |

| | | | | | |
|---|---|---|---|---|---|
| * 6.25 mg/PCR | | | | | |

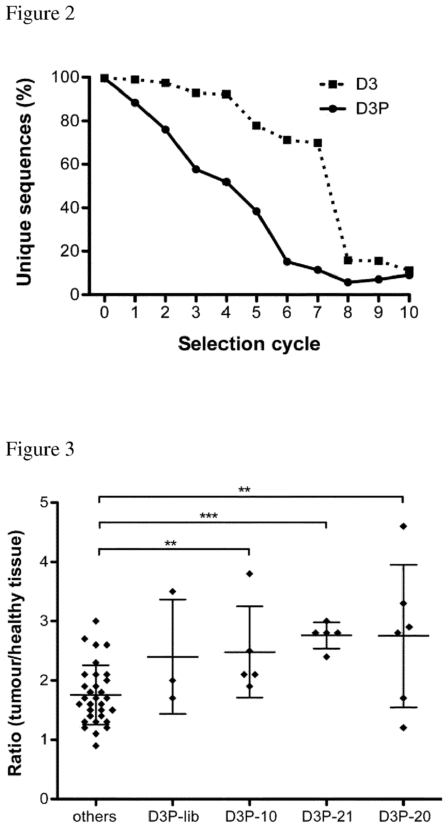

After ten *in vivo* selection cycles, each of the obtained DNA libraries was analysed by next-generation sequencing (NGS) as described above. Between 1.5·10⁵ and 1.2·107⁷ sequences per selection cycle were analysed from both selection procedures. The figure 2 shows the analysis of the number of unique sequences within the obtained libraries from the *in vivo* selection using D3 and D3P. As can be taken from the figure 2, the number of unique sequences was found to be significantly decreased, indicating both libraries being enriched. The PEG-modified DNA library D3P showed a steady decrease of unique sequences over the monitored DNA populations obtained from the different selection cycles. In contrast, the number of unique sequences in the naive DNA library D3 was strongly decreased from the selection cycle 7 to 8. This behaviour was also eminent from the distribution of the four nucleotides (dA, dG, dT, and dC) throughout the initial random region, which was similar up to selection cycle 4 and 7 of the libraries D3P or D3, respectively. In turn, the nucleotide distribution was clearly altered when analysing the DNA populations obtained after ten selection cycles, which already became evident in selection cycles 6 (D3P) and 8 (D3).

In the following and if not otherwise stated, the results refer to the DNA populations of both libraries obtained after 10 selection cycles. In a following step, individual sequences were chosen for further testing based on their enrichment profiles. In particular, collection was defined by i) copy number, *i.e*. the frequency of an individual sequence in a DNA population and ii) amplification fold, *i.e.* change of copy numbers from one selection cycle to another. Based on these criteria 46 sequences (22 from D3 and 24 from D3P) were selected for further assessment, among them 17 sequences with low copy numbers, *i*.*e.* frequency < 0.5% but enrichment profiles similar to those found for the most abundant sequences. The 46 sequences were subjected to an initial *in vivo* screening procedure using variants of the sequences bearing a near-infrared fluorescent dye (Alexa Fluor 680) at their 3'-ends. Sequences obtained from the library D3P were additionally equipped with a 5'- 11kDa PEG moiety. The sequences were evaluated using fluorescence reflectance imaging (FRI) of mice that bear orthotopic and subcutaneous xenograft tumours. The individual sequences were injected in the tail vein of anesthetised mice and whole-body FRI of the dorsal side view was performed before, 5, 60, and 180 minutes post injection. Subsequently, all animals were euthanised and several organs, including the orthotopic and the subcutaneous tumours, were harvested and analysed by ex *vivo* FRI. An initial screening using a single mouse per individual sequence was performed. Each sequence was evaluated for tumour targeting comparing the mean fluorescence inside the subcutaneous tumour to the mean fluorescence measured in a healthy zone adjacent to the tumour. None of the sequences derived from the library D3 were found to target efficiently the subcutaneous tumours.

Orthotopic tumour targeting was also assessed by *ex vivo* fluorescence measurements comparing the fluorescence of prostatic tumours vs. muscle. The fluorescence ratio between prostate tumour and muscle were higher than 2 for two sequences denoted D3P-10 (SEQ ID NO: 6) and D3P-21 (SEQ ID NO: 1). These two sequences and a third sequence denoted D3P-20 (SEQ ID NO: 7) revealed superior subcutaneous tumour targeting. Further testing of these sequences using additional subcutaneous mouse model samples revealed a reproducible tumour targeting when compared to the starting library and all other analysed sequences.

The figure 3 shows the validation of aptamers evolved from the DNA library D3P by planar imaging. As can be seen in the figure 3, the ratio of subcutaneous fluorescence signal obtained from the tumour tissue compared to surrounding tissue of all indicated sequences 180 min post injection. The values obtained from the starting library (D3P-lib) (n = 3), and the aptamers D3P-10 (n = 5), D3P-20 (n = 6), as well as D3P-21 (n = 5) were compared to all other sequences tested and pooled as "others" (n = 31). The aptamers D3P-21, D3P-P20 and D3P-P10 have a statistically significantly higher tumour to healthy tissue ratios (2.76 ± 0.09, 2.75 ± 0.49, and 2.48 ± 0.34, respectively) compared to the mice injected with the other aptamers (1.75 ± 0.09). In contrast, the difference obtained with the naive library (D3P-lib) (2.400 ± 0.55) was not significant. Statistical significance was calculated using Graphpad Prism 6 using an unpaired t test model assuming that all data have the same standard deviation (SD). ***P* < 0.01 and ****P* < 0.001.

Among them, the aptamer of SEQ ID NO: 1, which was denoted D3P-21, was found to be the most promising sequence as it showed an average fluorescence ratio of tumour to healthy tissue of 2.76 ± 0.09, which is significantly higher and reproducible compared to the average ratio obtained by all other sequences (1.75 ± 0.09).

### Example 2

### Quantification of aptamer localised to tumour tissue

For further validation, the amount of aptamer localised to the tumour tissue was quantified by quantitative PCR (qPCR). Both, orthotopic and subcutaneous tumours from mice treated with D3P-21 or the D3P-library were homogenised and the DNA extracted. The obtained DNA was then subjected to qPCR as described above.

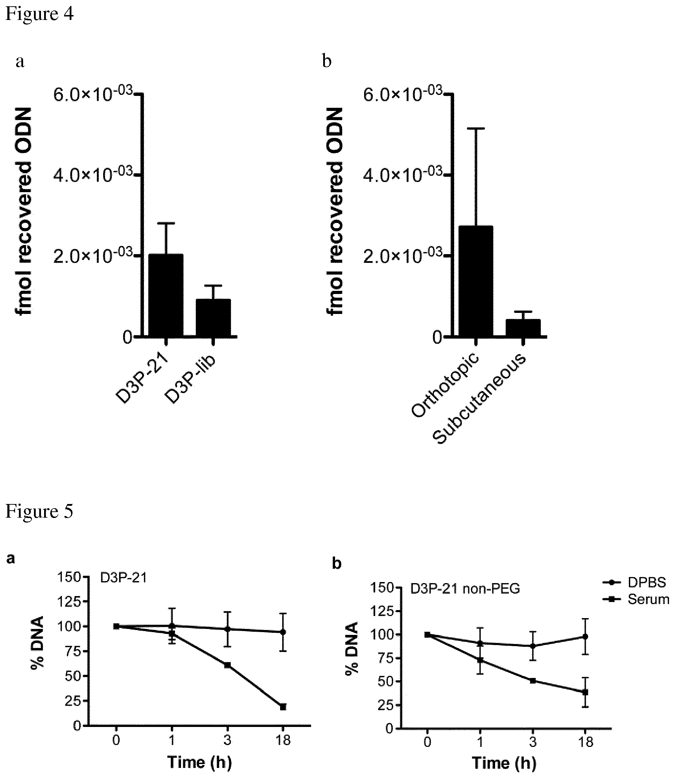

The figure 4 shows the quantification of tumour bindung of the aptamer of SEQ NO: 1, denoted D3P-*21, in vitro* in targeting subcutaneous or orthotopic PC-3 tumours extracted from mice used in the *in vivo* screening. The figure 4a shows the relative amount of the aptamer and native library (D3P-lib) used as control of tumours from only subcutaneous mouse models. The figure 4b shows the relative amount of aptamer D3P-21 from both orthotopic and subcutaneous tumours, both quantified *via* qPCR. Data was normalised to the OD of the sample and is represented as the mean value between tumours injected with the same oligonucleotide (n = 4, 2 independent experiments).

This analysis revealed a higher amount of D3P-21 recovered from subcutaneous tumours compared to the D3P-library. qPCR data also revealed that more copies of D3P-21 could be recovered from the orthotopic compared to the subcutaneous prostate tumour tissue. The heterogeneity of the recovered DNA amounts might be explained by the fact that the samples of the test set were non-perfused (in contrast to the samples directly obtained from the selection procedures), resulting in residual amounts of blood remaining in the tissue that interfere in the analysis.

### Example 3

### Determination of the stability of the aptamer of SEQ NO: 1

A pre-requisite for the potential therapeutic application of aptamers *in vivo* is their long-term stability in mammalian serum. As the aptamer of SEQ NO: 1, denoted D3P-21, was selected *in vivo,* this inherently indicates that the sequence has certain nuclease resistance. To further explore its stability, its degradation in serum and in Dulbeccos's phosphate buffer saline (DPBS) as a control were tested.

For testing the stability of the sequence, 2 µM D3P-21 aptamer either bearing or lacking the 11-kDa PEG moiety in the 5'-end were incubated in human serum and DPBS containing calcium and magnesium at 37°C. Samples were collected at different time points (0, 1,3 and 18 hours) and intact DNA was quantified with qPCR as described above. Human serum was kindly provided by Dr. Jens Müller from the University Hospital Bonn.

The figure 5 shows the stability of D3P-21 aptamer in human serum compared to DPBS with Ca²⁺/Mg²⁺ incubated in human serum (quadrants) or buffer (cycles). The figure 5a shows the stability of the pegylated aptamer, figure 5b shows the stability of the non-pegylated aptamer. As can be seen in figure 5, after 1 h of incubation at 37°C 92.8% of the pegylated aptamer remained full length in serum, which is -20% more compared to its non-pegylated variant (73.1 %). This difference becomes less significant after 3 hours of incubation, after which 61.1 % (D3P-21) and 50.8 % (non-pegylated D3P-21) of the respective full-length aptamers were detected. These data are in line with the qPCR results that also project towards a good stability of D3P-21 *in vivo.* While the PEG moiety seems to increase the endurance of D3P-21 towards nucleases up to 3h, extended incubation times revealed even more degradation of the pegylated variant compared to the non-pegylated aptamer. When incubated in DPBS, no degradation of the aptamer variants was observed.

### Example 4

### Determination of the binding properties of the aptamer of SEQ NO: 1 in vitro

The aptamer's characteristics and properties *in vitro* were analysed. To characterise the binding properties of the aptamer of SEQ NO: 1, denoted D3P-21, *in vitro,* flow cytometry studies were performed using PC-3 cells as described above. Further, the interaction of D3P-21 with other cancer cell lines, *i.e.* A459, H460, MCF-7, Ramos, and the androgen-dependent prostate cancer cell line LNCaP was tested. Furthermore, peripheral blood mononuclear cell (PMBC) and splenocytes from mice were also investigated. For technical reasons oligonucleotides (ODN, aptamer and controls) labelled with Atto647N fluorophore were used in the flow cytometry assay with LNCaP cells, while ODNs (aptamer and controls) labelled with Alexa Fluor 680 were employed for the other cell lines.

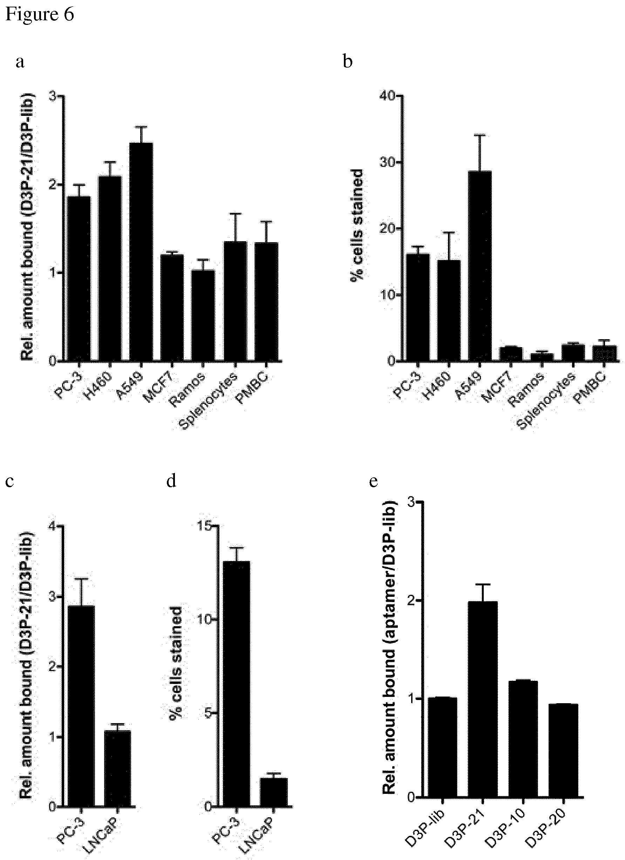

Figure 6 shows the results of the flow cytometry analysis of the interaction of aptamer of SEQ NO: 1 (D3P-21) and the DNA library D3P (D3P-lib) with prostate cancer PC-3 cells, murine splenocytes, and murine peripheral blood mononuclear cells (PBMC) and other cancer cell lines (MCF7, H460, A549, and Ramos). Figure 6a shows the ratio of binding of D3P-21 compared to the D3P-library and figure 6b the total percentage of cells bound by D3P-21. As can be seen from the figures 6a and 6b, the aptamer D3P-21 revealed a two-fold increase in binding to cultured PC-3 cells compared to the naive D3P library. Figure 6c shows the results of the flow cytometry analysis of the interaction of D3P-21 and D3P-lib with the prostate cancer PC-3 and LNCaP cells, wherein figure 6c shows the ratio of binding of D3P-21 compared to D3P-lib and figure 6d the total percentage of cells bound by D3P-21.

These experiments revealed that the aptamer of SEQ NO: 1, denoted D3P-21, interacts with the lung cancer cell lines A549 and H460 (lung carcinoma), while no binding to splenocytes, PMBCs, Ramos (Burkitt's lymphoma), and MCF7 (breast cancer) cells was observed. Notably, binding to androgen-dependent prostate cancer LNCaP cells was also not observed.

The figure 6e shows the results of the flow cytometry analysis of the interaction of aptamer of SEQ NO: 1 (D3P-21) compared to the aptamer candidates denoted D3P-10 and D3P-20 of example 1. As can be seen, the other aptamer candidates tested showed no binding to PC-3 cells *in vitro.* This experiment showes that the aptamer of SEQ NO: 1, denoted D3P-21, is the only aptamer interacting with cancer cell lines.

### Example 5

### Determination of the pegylation on the binding properties of the aptamer of SEQ NO: 1

The impact of the PEG moiety on the binding characteristics of D3P-21 was analysed. Therefore, PC-3 cells were incubated with pegylated (D3P-21), non-pegylated D3P-21 (D3P-21non-PEG), or the non-pegylated D3P-library (D3P-lib non-PEG) labelled with Atto647N at the 3'-end and the interaction of pegylated or non-pegylated D3P-21 with PC-3 cells was measured by flow cytometry and qPCR as described above and compared to the D3 and D3P libraries, respectively.

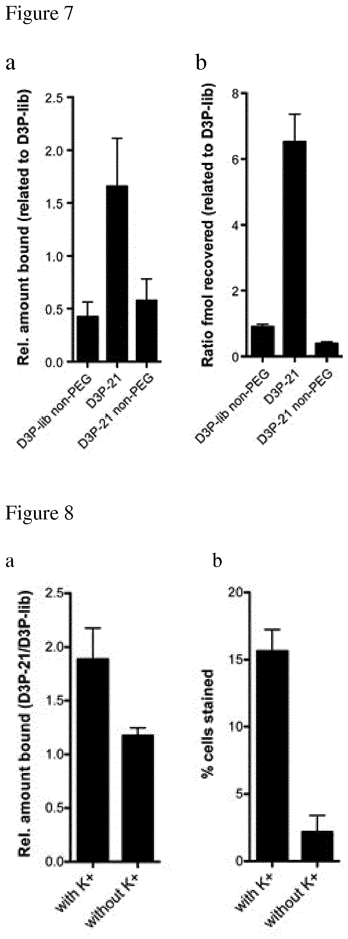

Figure 7a shows the results of the flow cytometry analysis of the impact of the PEG moiety of D3P-21 on its interaction properties. Figure 7b shows the analysis of the impact of the PEG moiety on D3P-21 on PC-3 cell interaction using non-labelled DNA and qPCR for quantification. Shown is the ratio of recovered fmoles of the indicated oligodeoxynucelotide compared to D3P-lib. Represented as mean ± SD (n = 4).

These experiments demonstrated a loss of binding performance of D3P-21 in the absence of the PEG moiety. Conversely, the binding of the D3 library to PC-3 cells was found to be independent of its pegylation status.

### Example 6

### Determination of the folding of the aptamer of SEQ NO: 1

The sequence of the aptamer of SEQ NO: 1 is a G-rich sequence (33.75%) and, thus, might be capable of folding into G-quadruplex structures. Although the aptamer does not share intersected runs of consecutive G residues, typically associated with G-quadruplex formation the dependence of cell binding of the aptamer on the presence of potassium ions were analyzed by flow cytometrie as these are key to G-quadruplex structure stabilisation. The cells were incubated with 100 nM of the aptamer labelled with Atto647N at the 3'-end as described above.

The figure 8 shows the flow cytometry analysis monitoring the influence of potassium in the binding of the aptamer of the aptamer of SEQ NO: 1, denoted D3P-21, to PC-3 cells. Shown is the ratio of binding of D3P-21 compared to D3P-lib in figure 8a and the total percent of cells bound by D3P-21 in figure 8b, represented as mean ± SD (n = 4). The figure 8 shows that the interaction of D3-P21 with PC-3 cells depends on the presence of potassium ions in the binding buffer.

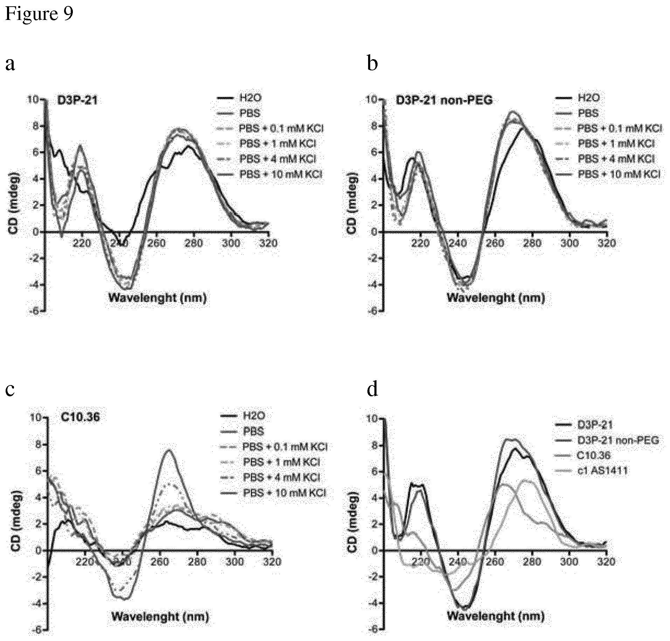

In order to elucidate whether D3P-21 forms a G-quadruplex structure and to analyse the impact of the PEG moiety on the conformation of D3P-21, circular dichroism (CD) spectroscopy experiments were performed as described above. The figure 9 shows the CD spectroscopy analysis of D3P-21. CD spectra of D3P-21 in figure 9a, non-pegylated D3P-21 (D3P-21 non-PEG) in figure 9b, and C10.36 in figure 9c under different conditions, *i.e*. H₂O, PBS w/o potassium ions (pH 7.4), and PBS with increasing concentrations of potassium ions as indicated. Comparison of the obtained CD spectra of different aptamers and variants at a concentration of 4 mM potassium ions in figure 9d.

As can be seen in the figure 9, the CD spectra of the aptamer were characterised by a positive peak at 273 nm (D3P-21) and 270 nm (non-pegylated D3P-21) and a negative peak at 244 nm. These typical CD profiles indicate that D3P-21 most likely contains a B-form conformation. Notably, the absence or presence of potassium ions did not have an impact on the CD spectra, indicating that the conformation of D3P-21 is K⁺-independent. In contrast, the CD spectra of C10.36, a previously described parallel G-quadruplex containing aptamer, were found to be strongly affected by the absence or presence of K⁺. Minor differences were observed in the CD spectra of the pegylated *vs*. the non-pegylated aptamer revealing that the 11 kDa PEG moiety has little or no effect on the aptamer's conformation.

These data also indicate, that the aptamer of SEQ NO: 1 most likely does not fold into a common G-quadruplex structure.

### Example 7

### Determination of a possible activate the innate immune system by the aptamer of SEQ NO: 1

The impact of D3P-21 on the innate immune system was evaluated since it was suggest that DNA aptamers activate the immune system, mainly mediated by the Toll-like receptor (TLR) superfamily. To determine D3P-21's potential in this regard the secretion of TNFα by immortalised murine embryonic stem cell-derived macrophages upon aptamer treatment was measured. Increasing concentrations of D3P-library, D3P-21, D3P-21 non-pegylated, CpG ODN 1826 type B and LPS were incubated with immortalized murine embryonic stem cell-derived macrophages for 24 h and concentration of TNF-alpha in the supernatant was determined by HTRF assay as described above.

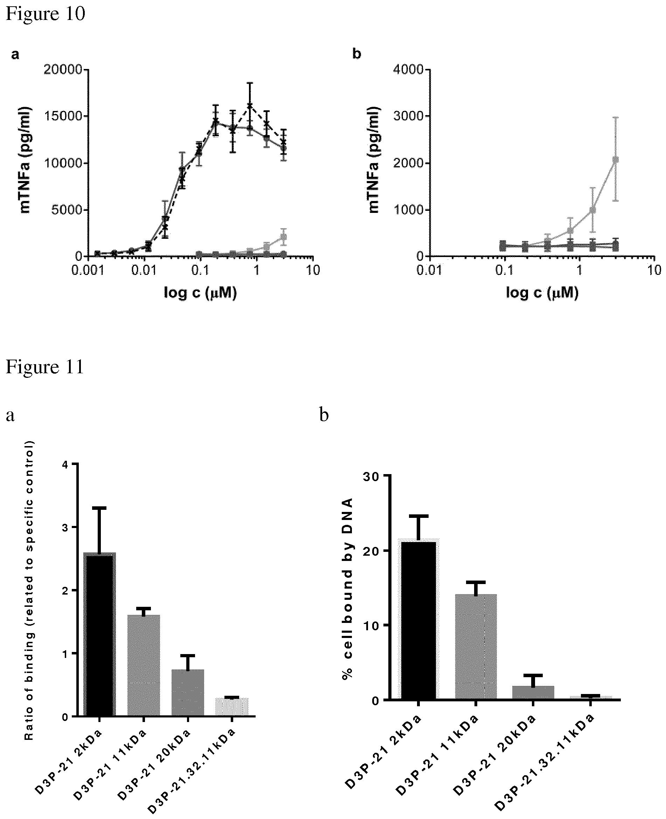

The Figure 10 shows the evaluation of aptamer immunogenicity. The Figure 10a shows concentration of TNF-alpha in the supernatant vs. the concentrations of D3P-library (grey sqares), D3P-21 (dots, below), non-pegylated D3P-21 (black squares), CpG ODN 1826 type B (dots, above) and LPS (x). The results of the D3P-library (grey sqares), the aptamer D3P-21 (dots), and non-pegylated D3P-21 (black squares) are depicted in Figure 10b without controls. The results are represented as mean ± SEM (n = 5).

As can be taken from the figure 10, the results demonstrated a very low immunogenicity by D3P-21 and its non-pegylated variant at concentrations up to 3 µM. In contrast, the D3P-library induced TNFα secretion at concentrations above 0.375 µM. This finding is not surprising as the initial library contains ∼10¹⁵ sequences, some of which most likely sharing structural motifs capable of TLR recognition. However, the activation of the innate immune response by the DNA library was found to be >3 orders of magnitude less pronounced compared to the controls, *i.e.* CpG and LPS.

These data indicate that the aptamer of SEQ NO: 1 does not activate the innate immune system. This provides a major advantage, as the lack of stimulating the innate immune system overcomes a major drawback linked to DNA aptamers.

### Example 8

### Determination of the influence of the molecular weight of the polyethylene glycol (PEG) moiety on binding

The dependence of cell binding of the aptamer on the weight of the polyethylene glycol (PEG) moiety was analyzed by flow cytometrie. The aptamer D3P-21 as selected in example 1 contained an 11kDa polyethylene glycol (PEG) moiety on the 5'-end. For comparison of (PEG) moieties, the aptamer sequence SEQ NO: 1 conjugated to an 2kDa and an 21kDa polyethylene glycol (PEG) moiety on the 5'-end, respectively, was used. PC-3 cells were incubated with 100 nM of the aptamers of SEQ NO: 1 with 2kDa, 11 kDa and 21kDa PEG moieties labelled with Atto647N at the 3'-end as described above. A non binding 32nt sequence having a 11 kDa PEG moiety (denoted D3P-21.32 11kDa, SEQ NO: 5) was used as control.

The figure 11 shows the results of the flow cytometry analysis monitoring the influence of the weight of the PEG moiety on the binding of the aptamer of SEQ NO: 1 to PC-3 cells. The aptamers of SEQ NO: 1 with 2kDa, 11 kDa and 21kDa PEG moieties are denoted D3P-21 2kDa, D3P-21 11kDa, D3P-21 22kDa, respectively. Shown is the ratio of binding compared to the starting library as "specific control" in figure 11a and the total percent of cells bound in figure 11b, represented as mean ± SD (n = 4). The figure 11 shows that the binding of the aptamer of SEQ NO: 1 to PC-3 cells decreased with increasing molecular weight of the polyethylene glycol (PEG) moiety.

In summary, these results show that the aptamer of SEQ ID NO: 1 comprising a polyethylene glycol moiety conjugated to the 5'-end is a tumour targeting aptamer with godd specificity, particularly to hormone refractory prostate cancer. The aptamer was able to interact with cultured PC-3 cells *in vitro,* and demonstrated the capacity to recognise a target that is present on the tumour cells in culture and in the tumour microenvironment *in vivo.* The aptamer also recognised lung cancer cell lines.

The work leading to this invention has received funding from BMBF under grant agreement n° 13N12249.

## Claims

1. An aptamer, wherein the aptamer comprises a nucleotide sequence as given as follows: 5'-GCTGTGTGACTCCTGCAAGGAAAGAGCACGGCCAAGTCAGGGGGAATCGACTAC GTCGGGGGGAGACAAGATACAGCTGC-3' (SEQ ID NO: 1) or a fragment thereof of at least 20 contiguous nucleotides of the nucleotide sequence as given as follows: 5'-GGAAAGAGCACGGCCAAGTCAGGGGGAATCGACTACGTCGGGG-3' (SEQ ID NO: 2), wherein the aptamer comprises a polyethylene glycol moiety conjugated to the 5' or the 3' end.

2. The aptamer according to claim 1, wherein the polyethylene glycol moiety has a molecular weight in a range of ≥ 0.1 kDa to ≤ 90 kDa, preferably in a range of ≥ 2 kDa to ≤ 20 kDa, particularly in a range of ≥ 2 kDa to ≤ 11 kDa.

3. An aptamer, wherein the aptamer comprises a nucleotide sequence as given as follows: 5'-GCTGTGTGACTCCTGCAAGGAAAGAGCACGGCCAAGTCAGGGGGAATCGACTAC GTCGGGGGGAGACAAGATACAGCTGC-3' (SEQ ID NO: 1) or a fragment thereof of at least 20 contiguous nucleotides of the nucleotide sequence as given as follows: 5'-GGAAAGAGCACGGCCAAGTCAGGGGGAATCGACTACGTCGGGG-3' (SEQ ID NO: 2), wherein the aptamer comprises a polyethylene glycol moiety conjugated to the 5' or the 3' end for use as a medicament or a diagnostic reagent.

4. An aptamer, wherein the aptamer comprises a nucleotide sequence as given as follows: 5'-GCTGTGTGACTCCTGCAAGGAAAGAGCACGGCCAAGTCAGGGGGAATCGACTAC GTCGGGGGGAGACAAGATACAGCTGC-3' (SEQ ID NO: 1) or a fragment thereof of at least 20 contiguous nucleotides of the nucleotide sequence as given as follows: 5'-GGAAAGAGCACGGCCAAGTCAGGGGGAATCGACTACGTCGGGG-3' (SEQ ID NO: 2), wherein the aptamer comprises a polyethylene glycol moiety conjugated to the 5' or the 3' end for use in the detection, diagnosis or treatment of cancer.

5. The aptamer for use according to claim 4, wherein the cancer is selected from lung cancer and prostate cancer, particularly hormone refractory prostate cancer.

6. The aptamer for use according to anyone of claims 3 to 5, wherein the polyethylene glycol (PEG) moiety has a molecular weight in a range of ≥ 0.1 kDa to ≤ 90 kDa, preferably in a range of ≥ 2 kDa to ≤ 20 kDa, particularly in a range of ≥ 2 kDa to ≤ 11 kDa.

7. A composition comprising an aptamer according to anyone of claims 1 or 2.

8. A pharmaceutical composition comprising as an active ingredient an aptamer according to anyone of claims 1 or 2.

9. The composition according to claim 7 or 8 for use in the detection or diagnosis or the treatment of cancer, particularly hormone refractory prostate cancer.

10. A cancer-specific drug delivery composition comprising an aptamer according to anyone of claims 1 or 2, and an anti-cancer agent such as a toxin, an anti-cancer growth inhibitor gene, an antagomir, siRNA, or combinations thereof.

11. Use of an aptamer comprising a nucleotide sequence as given as follows: 5'-GCTGTGTGACTCCTGCAAGGAAAGAGCACGGCCAAGTCAGGGGGAATCGACTAC GTCGGGGGGAGACAAGATACAGCTGC-3' (SEQ ID NO: 1) or a fragment thereof of at least 20 contiguous nucleotides of the nucleotide sequence as given as follows: 5'-GGAAAGAGCACGGCCAAGTCAGGGGGAATCGACTACGTCGGGG-3' (SEQ ID NO: 2), wherein the aptamer comprises a polyethylene glycol moiety conjugated to the 5' or the 3' end, for the manufacture of a medicament or a diagnostic reagent, preferably for the detection or diagnosis or the treatment of cancer, particularly hormone refractory prostate cancer.

12. An in vitro method of detecting or diagnosing cancer, the method comprising the step of detecting the binding of an aptamer according to anyone of claims 1 or 2 to a cell, tissue, or sample obtained from a subject.

13. A method of treating cancer, the method comprising the step of administering to a subject a therapeutically effective amount of an aptamer according to anyone of the claims 1 or 2, a pharmaceutical composition according to claim 8, or a cancer-specific drug delivery composition according to claim 10.

14. A method for selecting aptamers specific for a target sample, the method comprising the steps:
- providing a library comprising putative disease-specific aptamers;
- incubating the library with a target sample;
- washing the target sample to remove aptamers that are not bound;
- eluting the aptamers bound to the target sample,
- amplifying the eluted aptamers using polymerase chain reaction to generate an enriched pool of aptamers;
- repeating the incubating, washing, eluting and amplification steps a plurality of additional times to generate a pool of enriched aptamers that are specific for the target sample, and
- sequencing the pool of enriched aptamers to determine their sequence and relative frequency within the pool,
wherein the aptamers of the library comprise a conjugated polyethylene glycol moiety.

15. The method according to claim 14, wherein method is an *in vivo* method, preferably performed in a murine orthotopic xenograft model.
